# EUROPEAN PATENT SPECIFICATION

(11) **EP 4 038 362 B1**
(45) Date of publication and mention of the grant of the patent: **28.02.2024**
(21) Application number: 20788904.9
(22) Date of filing: 25.09.2020
(51) Int. Cl.: G01N 21/85, G01N 11/12, G01N 21/47, G01N 33/04, G01N 27/02

(54) **MEASURING SYSTEM FOR FOODSTUFFS**
MESSSYSTEM FÜR LEBENSMITTEL
SYSTÈME DE MESURE POUR DENRÉES ALIMENTAIRES

(30) Priority: 01.10.2019 NL 2023922
(43) Date of publication of application: 10.08.2022
(73) Proprietor: Lanvi Patent B.V., 3147 PB Maassluis (NL)
(72) Inventor: VAN HALSEMA, Frans Emo Diderik, 3147 PB Maassluis (NL)
(74) Representative: Octrooibureau Van der Lely N.V.
(86) International application number: PCT/NL2020/050596
(87) International publication number: WO 2021/066646

(56) References cited:
- WO-A1-2017/188462
- DE-C1- 19 920 928
- US-A- 4 448 060

## Description

The present invention relates to a measuring system for automatically determining and/or monitoring the quality of liquid or viscous foodstuffs, and comprising a housing with a space for accommodating at least one container for such a liquid or viscous foodstuff, at least one container for or with said foodstuff, with a probe which projects into the container and comprises a thermometer, a viscosity measuring device for determining a viscosity value of the foodstuff in said space, and a control unit which is configured to control the measuring system and which is operatively connected to the viscosity measuring device for repeatedly performing a viscosity measurement and for storing and/or exporting and/or processing the determined viscosity value, wherein the viscosity measuring device comprises a magnetic first body, a controllable electromagnetic drive for the first body, and a detection system for detecting displacement of the body, comprising a plurality of proximity sensors, in particular Hall sensors, wherein, in use, the first body is situated in the container.

Such a measuring system is known per se, for example from GB931471A for printing inks, and DE2204878 A1 and EP0036801 A1 for blood or other body fluids. A measuring system according to the preamble of claim 1 is known from US4448060. Relevant prior art can be also found in the disclosure of documents WO 2017/188462 A1 and DE 199 20 928 C1.

For a variety of reasons, such meters are often not very suitable for performing measurements on liquid or viscous foodstuffs. Firstly, such measurements on liquid or viscous foodstuffs require a great many measurements, because many methods of preparation have to be investigated when developing a (new) dairy product, and under many different circumstances. In particular, there is currently a shift away from the consumption of animal proteins towards the consumption of vegetable proteins. In order to provide vegetable alternatives to the respective animal-based, often dairy, products which have similar properties requires a large number of experiments.

It is therefore often desirable to be able to perform many measurements simultaneously and together in order to keep things well-organised, to ensure identical conditions for the various methods of preparation and tests, etc. Mutual influencing of measurements or measuring arrangements is undesirable in that case.

Now, many of the known "falling ball" systems use an electromagnet which attracts a remote body. This requires a relatively strong magnet, the stray field of which may disturb adjacent arrangements. Although screening is not impossible, such an additional and unnecessary effort is undesirable.

Furthermore, it is desirable to make the measuring system suitable for products with very diverse viscosities, such as milk, yoghurt, quark, ketchup, (cream) cheese, etc. It is not readily possible to ensure, on the one hand, a sufficient falling distance and, on the other hand, to keep the magnetic strength for attracting the body (for repeated measurement) through the product within limits.

Furthermore, the MQC-C measuring system of the Lely Astronaut^{®} milking robot is known. This comprises a chamber in which a milk sample is gelled and in which a body drops down after it has been raised electromagnetically, in which case a viscosity, and from that a cell number and from the latter the risk of mastitis is determined from the measured fall time. This system (thus) also has the same limitations and drawbacks as the abovementioned system.

It is an object of the present invention to develop a measuring system of the kind described in the introduction further which at least partly overcomes said limitations and drawbacks, at least to provide a suitable alternative.

The invention achieves this object with a measuring system according to Claim 1, in particular a measuring system for automatically determining and/or monitoring the quality of liquid or viscous foodstuffs, and comprising a housing with a space for accommodating at least one container for such a liquid or viscous foodstuff, at least one container for or with said foodstuff, with a probe which projects into the container and comprises a thermometer, a viscosity measuring device for determining a viscosity value of the foodstuff in said space, and a control unit which is configured for controlling the measuring system, and which is operatively connected to the viscosity measuring device for repeatedly performing a viscosity measurement and storing and/or exporting and/or processing the determined viscosity value, wherein the viscosity measuring device comprises a magnetic first body, a controllable electromagnetic drive for the first body, and a detection system for detecting displacement of the body, comprising a plurality of proximity sensors, in particular Hall sensors, wherein the first body, in use, surrounds the probe and is displaceable around the probe on account of the drive, wherein, in use, the first body is situated in the container, wherein the drive comprises a plurality of individually energisable coils which are wound around the container which are stacked in a stack, wherein the control unit is configured to individually energise the coils in a predetermined pattern, in such a way that the first body is displaced in the container.

By using a plurality of stacked electromagnets according to the invention instead of a single, remotely placed electromagnet, it is much more readily possible both to use a low magnetic strength, making it possible to use various arrangements and measuring systems in close proximity, and to control the displacement of the magnet body, thus also rendering it simple to perform many, and also reliable, measurements under the many different viscosity conditions of all kinds of liquid or viscous foodstuffs. In this case, it is, for example, possible to move the body up, but also down, by energising and de-energising the coils in the sequence of the stack.

When using the measuring system, the probe will project into the container right into the product/foodstuff in the product container. To this end, the probe may extend close to the bottom of the product container, so that it still provides correct results even if the latter is only partly filled. Obviously, for correct use, the product container always has to be filled to a level (well) above the thermometer and/or other sensors in or on the probe.

As regards the viscosity value(s), it should be noted here that various values may be assigned to the viscosity of a foodstuff, such as dynamic viscosity, kinematic viscosity and bulk viscosity, although these can usually be converted with respect to each other. However, what is more important is the fact that the viscosity/viscosities may depend on both velocity and temperature. According to the invention, it is possible to control both.

Specific embodiments are described in the dependent claims and in the following part of the description.

In embodiments, the pattern is adjustable. This offers the possibility to move the first body up or even actively down in a desired way, all this of course partly dependent on the resistance/viscosity of the product to be tested. It would also be possible to adjust the falling distance in this way, if it is not necessary or even undesirable if the first body rises or falls for the entire available height of the container.

In particular, the pattern comprises repeatedly energising successive coils in a reciprocating cycle. In this way, the contents of the container can be mixed efficiently, even in the case of a narrow container, such as a test tube or the like. Mixing the contents of such a test tube or other narrow container using conventional magnetic rotary stirrers is virtually impossible, because these only cover the bottom section. Obviously, it is nevertheless possible to alternatively or additionally provide a magnetic rotary stirrer.

In embodiments, the pattern comprises successively temporarily energising a coil and temporarily energising an adjacent coil after a delay time, in such a way that the first body is displaced in the space of the container at a velocity, wherein the control unit is configured to adjust at least one of the delay time and the velocity. As has already been indicated above, the first body can be pulled up or can also be pulled down in accordance with the sequence of the stack. It is also possible to control the velocity at which this up and/or down pulling movement of the first body is carried out. After all, in particular with highly viscous foodstuffs it may be important to ensure that the velocity at which the viscosity is measured is not too high or too low, while it is exactly this high or low viscosity which can have a significant effect on this fall velocity. Additional "support" for the fall by pulling down the electromagnets can then increase the accuracy and/or the reliability of the measurement. In addition, depending on the velocity through the foodstuff, there may be various viscosity values involved, for example because the type of flow around the first body changes. In this case as well, it is useful if this velocity can be adjusted by means of the measuring system according to the invention.

In particular, the plurality of proximity sensors are provided on an elongate carrier in the container, more particularly on the probe. Alternatively or additionally, the plurality of proximity sensors are provided between the container and the plurality of coils. In the first case, the sensors are on a separate carrier or on the probe and this in the container. The distance between the first body and the proximity sensors can then be made very small, so that the measurements can, in principle, be very reliable. On the other hand, the sensors are then always exposed to the product and cleaning may be problematic, so that the embodiment in which the sensors are situated outside the container offers the corresponding advantages.

In particular, the plurality of proximity sensors are placed at a mutual distance apart which, in use, increases in a downward direction. This makes it possible to ensure that the time spans when passing the various successive sensors do not become too short at a low viscosity, whereas, by contrast, many different fall time measurements become possible at a very high viscosity. In all cases, this ensures an accurate measurement of the velocity and consequently an accurate determination of the viscosity.

In embodiments, the measuring system is furthermore provided with a cleaning device, comprising a magnetic second body which, in use, is provided in the container under the first body and is displaceable on account of said coils, and is provided with a cleaning part on an outer periphery which bears against an inner wall of the container, wherein a stop is provided on the carrier and/or on said inner wall of the container for cooperation with the second body, which stop determines a maximum elevation for the second body which is lower than a maximum elevation for the first body. In some cases, it may be advantageous to be able to clean at least the inner wall of the container, such as removal of deposit of the often protein-rich product. To this end, a movable magnetic second body is provided with one or more cleaning parts, such as rubber brushes, which, in use, bear against the inner wall. By displacing the second body by means of the coils, these brushes or the like clean the inner wall. The stop in the form of, for example, one or more small lugs ensures that the second body can easily be separated from the first body, although other measures to that effect are not excluded.

In particular, the measuring system furthermore comprises a magnetic drive for the second body and/or the first body which is provided under or at the bottom of the container. This measure, which is known per se, serves as a (magnetic) stirrer in the container and works better if the container has a smaller height/width ratio, as has already been indicated above.

In embodiments, the measuring system furthermore comprises a conditioning unit for conditioning said space of the housing, comprising a heating and cooling device, and operatively connected to the thermometer. In particular with liquid or viscous foodstuffs, and to a lesser degree with any other edible or other products, measuring the behaviour and the properties at different temperatures is of utmost importance. Therefore, the measuring system according to the invention offers significant advantages, as it is suitable for automatically measuring and/or monitoring the properties of many products under many different conditions, in particular temperatures.

In particular, the control unit is configured to control the conditioning unit, comprising causing the space of the housing to undergo a predetermined time-temperature program. The time-temperature program comprises, in particular, bringing the space in the housing to a desired temperature, optionally at a desired temperature profile, and thus indirectly the container and its contents, and subsequently keeping it at this desired temperature for a predetermined time. Likewise optionally is the subsequent cooling of the space, for example forced cooling, again in particular according to a desired temperature profile. If desired, these steps may be repeated one or more times or may contain even more steps, such as a repetition, but at different temperatures or heating and or cooling in a plurality of steps for, optionally, different lengths of time. All this mimics the temperature variations which often occur during use of a foodstuff, such as a dairy product, be it during production, transportation or storage (in a shop or at home), but also during use, if the contents of a container are not finished in one go. This is of particular importance in the case of products which are to be refrigerated, such as dairy products, because these are usually located in a much warmer space during use and will therefore be subject to decay more quickly.

In this case, the heating and cooling device is obviously in heat-conducting contact with the housing or the or each container, and the thermometer in the container is used by the control unit to measure a temperature of at least the container, at least a temperature which is representative for a content of the container, and thus for controlling the heating and cooling device.

Said embodiments are highly suitable for automatically, that is to say without the interference of error-prone humans, performing the great number of measurements which are required to examine large numbers of (dairy) products with different recipes in a search for a new or improved (dairy) product. In this context, reliability and repeatability are of great importance, so that automatic measurements with the measuring system according to the invention may provide significant advantages.

In particular, the cooling system comprises a coolant circuit which is in heat-conducting contact with the container or a number of containers which is adjustable by the control unit by means of a valve block which is controllable by the control unit. The coolant circuit comprises a pump device and a coolant which is pumpable by the pump device and can be cooled down by the cooling system, such as advantageously by a Peltier cooling system, which does not have any moving parts. A drawback of a Peltier cooling system may be that the cooling capacity is limited, which may in turn be disadvantageous for correctly testing, in particular, liquid or viscous foodstuffs. After all, it is in particular the behaviour of proteins in these which is greatly affected by temperature, so that it may be important to reduce a higher temperature which has been maintained for a certain time quickly to a desired end temperature. In this case, a great cooling capacity is useful. Therefore, a cold buffer is advantageously provided, comprising a phase change material which undergoes a phase change, at least is able to store latent heat, at the desired end temperature. A known example is water, which melts/solidifies at 0°C, or a lower temperature if additives have been added, and has a great cooling capacity as an ice (water) bank. However, in many cases the temperature of 0°C is too low. In addition, it may be a large drawback of water as phase change material that it expands considerably when frozen. Other materials which are usable, such as in order to arrive at below the denaturation temperature of proteins, are, for example, all kinds of paraffins and glycerine. The cold buffer which is thus produced may be cooled by the cooling system, such as the Peltier cooler, for a (much) longer time, such as during the hot part of tests. Advantageously, in order to cool the container (or each or one thereof), the control unit is then configured to control the pump device so as to cause the coolant to flow along the cold buffer and subsequently along the container.

In specific embodiments, the cold buffer comprises a heat-conducting foam, in particular a metal foam, such as aluminium foam, which is at least partly filled with the phase change material. Many phase change materials, in particular the organic ones, such as paraffins and glycerine, have a relatively poor heat-conducting capacity, so that their cold buffer action might be limited. By providing the material in a metal foam which conducts the heat much better, this drawback is largely overcome.

In addition to viscosity, many other properties of the product (liquid or viscous foodstuff, in particular dairy product) may play a part when assessing the product. Therefore, one or more other sensor devices are advantageously provided, which determine one or more other parameter values of the product in the container. In embodiments, the container comprises an optical sensor device for determining a quality-related optical parameter value of the product in said space of the container. Consideration may be given to colour, transmission and diffusion of light by or in the product, and then in particular also to changes over time and/or on account of temperature. For example, a change in colour, a drop in transmission or an increase in diffusion may indicate an undesired chemical change, growth of bacteria and/or formation of deposits or the like.

In particular, the optical sensor device is configured and arranged to project into the space of the container, and the sensor device comprises a sensor housing with electrical and/or electronic connections, a light source for emitting light, a first light conductor with a first injection surface coupled to the light source, for collecting at least a portion of the light emitted by the light source, and for passing it through the first light conductor to a first ejection surface and from there emitting at least a portion of the collected light in a spatial emission angle in the container, at least one second light conductor with a second injection surface placed in said spatial emission angle, for collecting at least a portion of the light emitted by the first light conductor and passed through the space of the container and passing it through the second light conductor to a second ejection surface, a detector for the or each second light conductor for detecting the light ejected by the second light conductor. Thus, a sensor device is provided which can easily be introduced into the (dairy) product and perform the optical measurements there, without it containing any electr(on)ic components. In this case, it is assumed that the second injection surface is placed in a functional way, that is to say in such a way that the light transmitted by the product can actually be injected in the second light conductor, i.e. at an angle which is smaller than or equal to the critical angle. Advantageously, the first ejection surface and the second injection surface are in parallel planes and more advantageously a perpendicular bisector of the first ejection surface and a perpendicular bisector of the second injection surface substantially coincide. Examples of the measurements are a colour or spectrum measurement, and a transmission measurement, all also as a function of time and/or temperature.

In embodiments, the optical sensor device furthermore comprises at least one third light conductor with a third injection surface placed outside said spatial emission angle, for collecting and passing through the third light conductor to a third ejection surface of at least a portion of the light emitted by the first light conductor and diffused in the space of the container. By placing the third injection surface outside the spatial emission angle (or the emission cone) of the first ejection surface, the third light conductor can only collect light diffused by the dairy product.

In particular, the first ejection surface and the second injection surface each make an angle of virtually 45° with the respective longitudinal axis. In embodiments, after mirroring in the first ejection surface, the longitudinal axis of the first light conductor and, after mirroring in the second injection surface, the longitudinal axis of the second conductor substantially coincide. These are measures to easily ensure that the ejected light beam can also be injected efficiently, in which case a very compact sensor device can also be obtained.

Advantageously, one or each light conductor comprises an optical fibre having a respective longitudinal axis. Due to the advantageous properties (no reflection loss on the outer wall due to entirely internal reflection and very small absorption possible), optical fibres, such as glass fibres, PMMA fibres, etc. are a very useful option. However, the option to use hollow conductors which are internally mirrored is not excluded. Due to the often small distances, absorption is limited with reflection and the injection angle is much larger than with optical fibres. In particular, the respective longitudinal axes of all light conductors run parallel.

In embodiments, the light source comprises at least a portion of an integrating sphere with the first injection surface and at least one LED, and in particular a plurality of different LEDs, placed therein. An integrating sphere is a (more or less) spherical body which is coated on the inside with a (highly) reflecting layer, usually diffusely reflecting. If lit on the inside, such a sphere can homogenise this light well. This offers the possibility to uniformly inject light of an LED, but better still of a plurality of LEDs, ensuring that the light of all LEDs is injected more or less uniformly. For example, two or more LEDs are provided, each of which emit light in a different wavelength band, and all are controllable by the control unit. Thus, a very variable light source is provided which is able to inject the various kinds of light in the (first) light conductor(s) and is thus able to determine the transmission (and also absorption) as well as, optionally, the diffusion for the various wavelength bands or colours.

In embodiments, the sensor device furthermore comprises at least one of a camera, advantageously placed under the container and directed upwards towards the container, and a second measuring device for measuring conductivity or other electrical properties. The camera can also, that is to say additionally or alternatively, perform colour measurements and determine if sedimentation occurs, and/or other optical properties change. Obviously, in that case, the container is provided with a transparent window, in particular on its underside.

Measuring electrodes, and in particular, but not exclusively, configured for determining the relative permittivity, advantageously as a function of the frequency of the electric field created between the electrodes, are useful as a second measuring device for measuring conductivity or other electrical properties. This provides the possibility for electrochemical impedance spectroscopy, which may provide additional information about a property/properties of the product and about changes over time in this property/these properties.

In important embodiments, the measuring system comprises a plurality of housings, each with a space for or provided with a container for or with a liquid or viscous foodstuff, wherein the plurality of housings are mechanically couplable to each other. Optionally the plurality of housings are thermally connectable to the conditioning unit, with the control unit being configured to control the conditioning unit according to the same or different simultaneous desired time-temperature programs for at least two of the plurality of housings. Such a plurality of mechanically coupled housings which can both be heated and/or cooled according to a program, in particular regulated separately from each other, offer the advantage that the many required measurements can be designed to be compact and energy-efficient. In this case, the heating device may for example comprise different heating elements which are individually energisable by the control unit and the cooling system may comprise individual proportional valves to control the degree of cooling.

In particular, each housing comprises an independently openable and closable lid and, in particular, at least one sensor device is provided for each container. This offers the possibility to exchange or examine one or more containers and/or the product contained therein, such as for sampling. The sensor device is preferably provided in the lid so as to be click-fittable.

The invention will now be described in more detail by means of some exemplary embodiments and the drawing, in which:
Figure 1 diagrammatically shows a perspective view of a measuring system 1 according to the invention,
Figure 2 shows a diagrammatic and partly cut-away perspective view of a portion of a module 2 of the measuring system 1 according to the invention,
Figure 3 diagrammatically shows a perspective sectional view of a portion of a measuring probe 7,
Figure 4 shows a detail view of optical components of the measuring probe 7 from Figure 3,
Figure 5 diagrammatically shows a cross section through a measuring probe 7' according to the invention,
Figure 6 diagrammatically shows a perspective view in partial cross section of the measuring probe 7' from Figure 5, and
Figure 7 diagrammatically shows a perspective view of the first body 61.

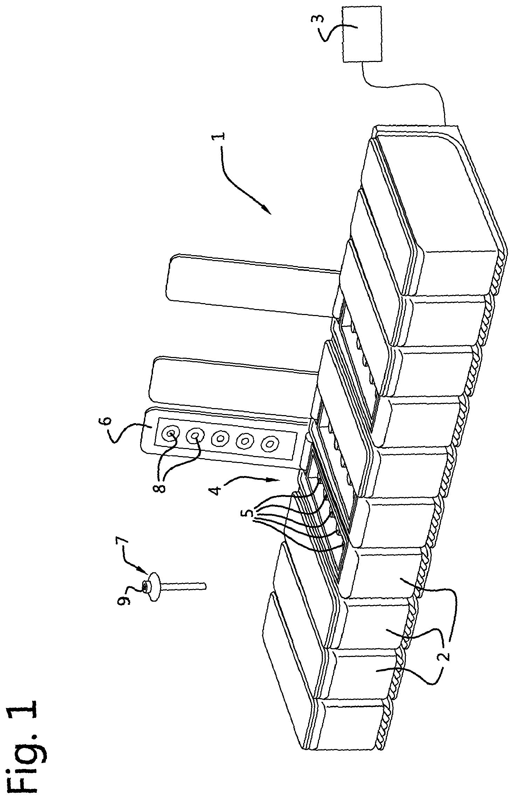

Figure 1 diagrammatically shows a perspective view of a measuring system 1 according to the invention, with ten modules 2 and an external control device 3.

Each module 2 has a space 4 for accommodating containers 5 and a lid 6. Reference numeral 7 denotes a measuring probe and reference numerals 22a and 22b denote electrical contacts and countercontacts, respectively.

The measuring system 1 illustrated here has ten modules 2, but may have any desired other number of modules, such as 1, 2, etc. This number can advantageously be changed during use. Furthermore, each module 2 here comprises a space 4 in which five containers 5 are arranged. The number of containers can also be chosen freely from 1, 2, etc., in which case the amount of space available in the space 4 has to be taken into account, for example by matching the dimensions of the containers 5 thereto.

Each container 5 can accommodate a foodstuff, for example a dairy product such as milk, yoghurt, quark, etc., but also any other low-viscosity or high-viscosity foodstuff, such as a fruit juice or fruit nectar, etc. After the container 5 has been filled, a measuring probe 7 can be introduced which is provided with one or more measuring instruments and the like. These may be operated and read out via electrical contacts. During closing of the lid 6, the countercontacts 22b are pressed against the contacts, following which the measuring probe(s) are in communication with, for example, the control device 3, and can then perform measurements. These measurements may be of many different kinds, as will be explained below later. The measurement data can then be exported to the external control device 3, where they may, for example, be stored, displayed and/or processed. Incidentally, the control device 3 may also be provided as an integral part of the measuring system, for example distributed over the modules 2. Furthermore, the control device may serve to control the measurements, for example to determine the moment of measuring. This will also be explained below in more detail.

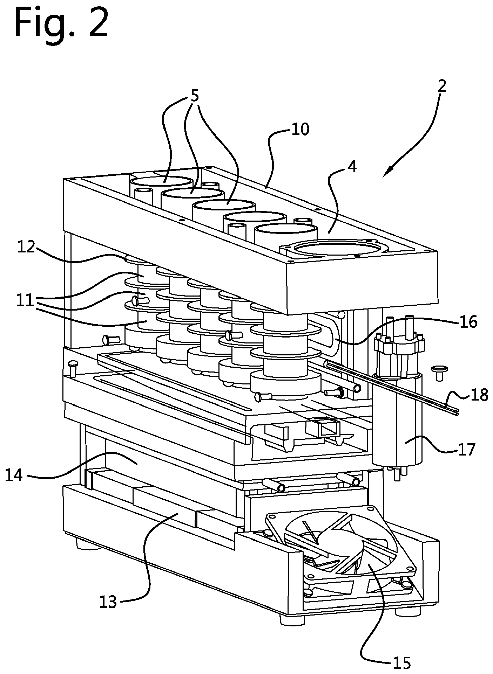

Figure 2 shows a diagrammatic and partly cut-away perspective view of a portion of a module 2 of the measuring system 1 according to the invention. The module 2 has a housing 10 containing the space 4 for the containers 5. A number of coils 11 are arranged around each container 5 between partitions 12. Reference numeral 13 denotes a Peltier cooling system, which cools a buffer vessel 14, while reference numeral 15 denotes a ventilator. A cooling circuit 16 is fed by means of pump 17, whereas reference numeral 18 denotes a connection for a heating system.

The coils 11 are individually electrically energisable and serve to displace a magnetisable body (not shown here) in the container 5 while measuring the viscosity of the product in the container 5. All this will be explained in more detail further below.

The product in the container 5 or the products in the various containers 5, respectively, may be conditioned by means of the conditioning unit, which comprises a heating system and a cooling system. Heating is provided (for example) via the connection 18 in the form of electrical heat. It is obviously possible to provide heat in a different way, but electrical heat has the advantage that it is readily and quickly controllable by means of a thermometer (not shown here). It should be stressed here that the various containers in one module may either be heated to the same temperature or, and advantageously, to different temperatures, by controlling the heating connection 18 differently, such as different heating coil density or a circuit with a different and adjustable PWM, etc.

In order to cool the product in the container(s) to a desired end temperature, two cooling systems are provided here. One cooling system comprises a cooling circuit 16 in which a coolant, such as glycol or water, is pumped around by means of the pump 17. Furthermore, a heat exchanger (not shown) is provided, such as cooling fins, in order to dissipate the heat taken up to the open air or the like. Optionally, however, a second cooling system is provided and in particular in the form of a selectable path of the cooling circuit via a buffer vessel 14. This contains a buffer for refrigeration to a desired (end) temperature in the form of a phase change material which has been cooled by means of the Peltier cooling system 13, at least latent heat has been withdrawn. To this end, for example, paraffin, water or the like has been converted from the liquid phase to the solid phase. Other phase transitions are not excluded. By subsequently switching a valve or the like in the cooling circuit 16, the coolant in the cooling circuit can be passed along or through the buffer vessel 14 in order to dissipate more heat there and to assume the (melting or at least phase transition) temperature of the buffer vessel more rapidly and for a prolonged period of time. In this way, forced cooling is possible without requiring a great deal of power, in this case using a Peltier cooling system 13 without moving parts but having the other associated advantages.

Furthermore, the control device of the measuring system, such as the external control device 3 from Figure 1, is advantageously configured to provide a desired temperature profile in the or each module 2. This temperature profile may comprise different temperatures for the various containers 5 (and thus for the products contained therein) in the module 2, but also a temperature-time profile, in which the temperature is specified as a function of time. For example, it is thus possible to examine the behaviour of and the changes in a product under specific thermal conditions. Thus, a product may be left unrefrigerated in the sun during transportation for some time or, for example, it may be removed from a refrigerator repeatedly for use on a table, etc. In this case, the prevailing temperatures are different every time. In order to keep the measurements "clean", a forced cooling system offers advantages, due to the fact that it excludes lagging effects of cooling down at different speeds as much as possible. However, this forced cooling by means of the cold buffer vessel 114 is optional.

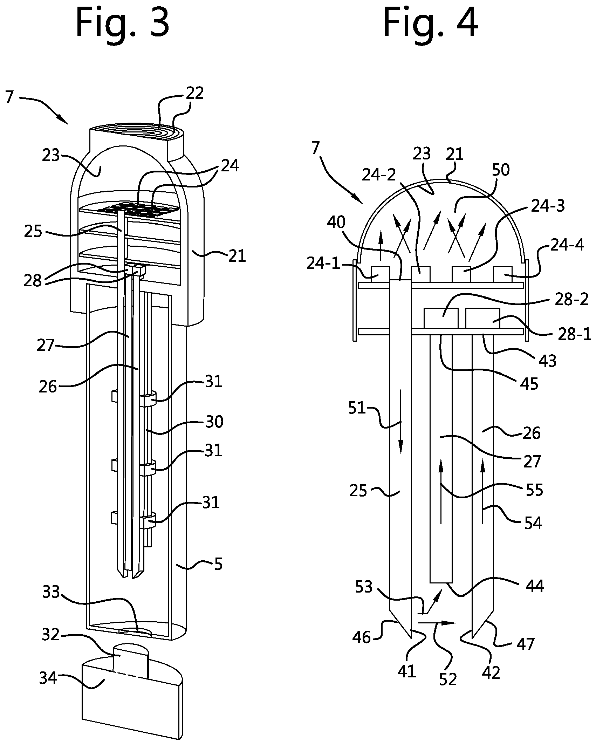

Figure 3 diagrammatically shows a portion of a measuring probe 7 in a perspective sectional view. The measuring probe 7 comprises a cover 21 which, in the closed position, adjoins the container 5 and which is provided with electrical connections 22. On the inside, a reflecting layer 23 has been provided, as well as LEDs 24.

Reference numerals 25, 26 and 27 denote a first, a second and a third light conductor, respectively, reference numeral 28 denotes two light detectors and reference numeral 30 denotes a mounting plate, on which there are three EIS (electr(ochem)ical impedance spectroscopy) electrodes 31.

A camera 32 looks through a window 33 and communicates with camera control unit 34.

In this case, the measuring probe 7 comprises some measuring devices for measuring different properties, each of which are optional per se. For example, optical properties, such as transmission and diffusion, are measured. To this end, light is injected in the product in the container. This is performed by means of LEDs 24 which inject light in the first light conductor 25 via the reflective layer 23, following which transmitted light is injected in the second light conductor 26, and diffused light into the third light conductor 27. All this is explained in more detail in Fig. 4.

Furthermore, EIS electrodes 31 are optionally provided on a plate 30. By means of the EIS electrodes, dielectric permittivity spectra or electrochemical impedance spectra of the product in the container 5 are determined in a manner known per se. Therefore, reference should be made to the prior art for details relating to the EIS measurements. An advantage is that these spectra can be determined for many products, but in particular also for many conditions, such as temperatures and temperature-time profiles.

The optional camera 32 offers the possibility of obtaining a visual or other optical image of the product. This may be particularly advantageous in order to monitor if changes occur, such as for example as a function of the time, the temperature, and/or the temperature-time profile. The changes may consist of a change in colour, changing transparency/turbidity, formation of depositions, etc. The camera control unit 34 advantageously comprises image-processing software. However, it is also possible to collect simple images with the camera 32 and to send these to an external processor via the camera control unit 34.

The communication from the measuring probe 7 to the "outside world" takes place, in particular, via the electrical connections 22, for example for the sake of energising the LEDs 24, reading out/actuating the detectors 28, the EIS electrodes 31 and the camera 32/the camera control unit 34, and any other components which have been provided.

Figure 4 provides a detail view of optical components of the measuring probe 7 from Figure 3. In this figure, and in the entire drawing, identical or similar components are denoted by the same reference numerals.

The measuring probe 7 comprises four LEDs, here a red LED 24-1, a green LED 24-2, a blue LED 24-3, and an infrared LED 24-4. During use, they emit light, which is denoted by reference numeral 50, and which is reflected by layer 23 on the inside of the cover 21. A portion 51 of the light 50 is injected in the first light conductor 25 via the first injection surface 40, is reflected by surface 46 and ejected via the first ejection surface 41. The ejected light is partly transmitted by the product as transmission portion 52, and captured and injected in the second light conductor via the second injection surface 42 to form portion 54, which is detected by first detector 28-1. The ejected light diffuses for another portion 53 in the product and is captured and ejected in the third light conductor 27 via the third injection surface 44 to form 55, which is detected by the second detector 28-2.

At least on the inside, the cover 21 is virtually semispherical and provided with a (diffuse or otherwise) reflective layer, such as magnesium oxide or barium sulfate, or gold, in particular if infrared measurements have to be performed. Thus, the (inside of the) cover is an integrating sphere which will evenly distribute the emitted light 50 for the sake of an even injection in the first light conductor 25. Incidentally, other injection methods, and the associated construction of the measuring probe 7, are not excluded. In this example, said light is emitted by the LEDs 28-1 to -4, being red, green, blue and infrared, respectively. However, any other light source or colour distribution/number of colours is also possible, such as specific colours, which may also be produced by lasers, or wide-band sources, such as halogen lights, etc. However, LEDs have advantages, such as compactness, long service life, high efficiency and availability in many colours with a relatively small bandwidth. LEDs 28-1 to -4 may be actuated separately from one another, so that no undesired influencing of the detectors 28-1 and -2 can occur.

For a more detailed explanation of the operation, only the red LED 28-1 is considered here, but a similar explanation applies to the other LEDs. The red LED 28-1 is actuated by the control unit (not shown here and, for example, external) in a desired pattern, such as once a minute. The emitted light reflects diffusely on the reflective layer 23 and will land relatively homogenously on the first injection surface 40 of the first light conductor 25. A portion 51 will be injected therein.

In this case, the first light conductor 25 is an optical fiber, such as a glass fiber or plastic fiber, as are the second and third, 26 and 27, respectively. These serve to transport the light by means of total internal reflection, so that losses are limited to the (small) absorption losses. However, in view of the mostly small distances, it is also possible to use a hollow, internally mirroring tube or a tube of transparent material which has been made reflective on the outside as a light conductor. An advantage of the latter is that more light can be injected, since the limitation of the critical entrance angle no longer applies.

The portion 51 which is injected reaches the surface 46 which is at virtually 45 degrees with the longitudinal direction of the first light conductor 25 here, and will then, in use, exit substantially horizontally from the first ejection surface 41, as light portion 52 and light portion 53, or light which is transmitted or diffused by the product in the container, respectively. The transmitted portion 52 reaches the second injection surface 42 of the second light conductor, and a portion of the injected light passes on, after mirroring on the surface 47, likewise placed at virtually 45 degrees, as portion 54 to the second ejection surface 43. There, the exiting light is detected by the light detector 28-1, as an indication for the transmission properties of the product.

Another portion of the light, portion 53 is diffused in the product, and can reach the third injection surface 44 of the third light conductor 27. It should be noted that it is precisely due to the use of the relatively limited critical injection and thus also ejection angle of optical fibers, that it is easy to prevent the third light conductor 27 from injecting direct and thus transmitted light, by placing the third injection surface 44 beyond the critical exit angle of the first light conductor 25. The light injected in the third light conductor 27 will reach the third ejection surface 45 as portion 55, and will be detected there by the light detector 28-2, as an indication for diffusion properties of the product.

By means of the illustrated embodiment, light can be injected in the product in an elegant way, with both the sources and the detectors and the control unit remaining outside the product. Obviously, other optical measuring methods also remain possible, such as when the LEDs 24, or other sources, are placed around the outside of the container, with the associated detectors also being situated around the container, so that the light passes through the entire container and the product. In particular with optically very dense products, such as dairy products, the latter barely makes sense, however.

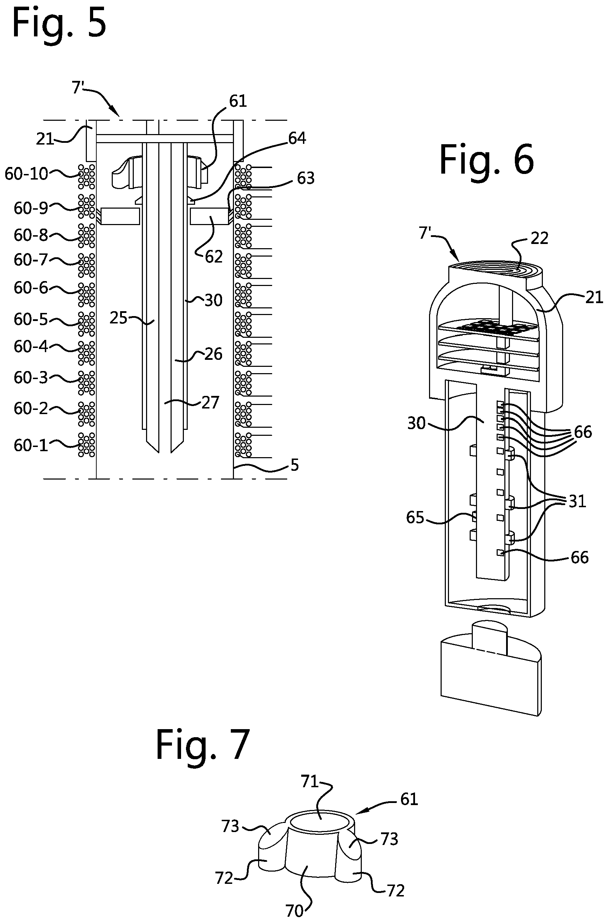

Figure 5 diagrammatically shows a cross section through a measuring probe 7' according to the invention. In this case, it comprises a plate 30 next to which light conductors 25, 26, 27 are arranged. Furthermore, reference numerals 60-1, ..., 60-10 denote ten coils around the container 5 and reference numerals 61 and 62 denote a first and second body, respectively, whereas reference numeral 63 denotes brushes and 64 denotes lugs.

The coils 60-1 to 60-10 are individually energisable via their respective connections, on the right in the figure, by means of an external power supply (not shown), under control of the control device, likewise external and not shown here. The magnetic field generated by the coil(s) attracts the first body 61 which is at least partly made of magnetic material, such as a permanent magnet or iron. When successively energising the bottom coil 60-1, then de-energising it and energising the penultimate coil 60-2, etc., the first body 61 can be pulled upwards. In this case, it should be noted that, in principle, any number of coils 60-xxx may operate satisfactorily, with more coils ensuring a smoother movement.

When it arrives at the top, the first body 61 can start a falling movement through the product in the container 5 by de-energising all coils. This falling movement may be detected by means of proximity sensors, in particular Hall sensors. In this context, see Figure 6 and the description thereof.

An advantage of this stepped upward drive, compared to the "shooting" upwards by means of a single coil, which generates a sudden strong field, is the fact that the velocity in the product can be limited and controlled. Particularly dairy products can have a very high viscosity, so that such a "launching" cannot work well, at least is not readily controllable.

It should furthermore be noted that, in particular with very high viscosities, it is also possible to attract the body by means of the coils. With a very low fall velocity, the (proximity/Hall) measurement often becomes inaccurate. It may then be helpful to increase this velocity by pulling the first body 61 using one or more additional coils. The magnetic force then has to be added to the force of gravity for the calculations. In this way, the viscosity can be determined with greater accuracy and in particular for a greater dynamic range, that is to say the viscosity as a function of the (end) velocity.

The illustrated first body 61 may furthermore serve as a stirrer, for example driving the magnetic material with a magnetic drive (not shown), which is known per se for magnetic stirrers.

Furthermore, a second body 62 is shown which is also at least partly made of magnetic material and can thus be moved up and down by energising the coils 60. The second body also comprises brushes 63, advantageously made of a flexible material, such as rubber or the like. The cross section of the second body 62 with the brushes 63 is such that the brushes 63 can clean the inside of the container 5 by friction when moving up and down. To this end, the coils 60 can move the second body 62 up first, such as more or less together with the first body 61. However, while the first body 61 can move upwards completely, that is to say as far as the top coil 60-10, the second body 62 is only able to rise as far as the lugs 64. As a result thereof, the first body 61 and the second body 62 can be separated mechanically. By now holding the first body 61 securely with the top coil 60-10, and de-energising the other, or energising even lower coils, the second body will move down. In this case, "pulling" using correspondingly energised coils will aid cleaning, in particular when the frictional force is great, which is fundamentally desired when cleaning. In this way, the second body 62 can be moved to and fro a few times by switching the coils 60 on and off in a corresponding way. Incidentally, the first body 61 and the second body 62 can also already be separated at the bottom by keeping the bottom coil 60-1 energised while energising the higher coils 60-2, 60-3, ... With suitable dimensions, in particular the height, of the second body 62, the first body 61 will then be attracted more by the higher coil 60-2 than by the bottom coil 60-1, while the opposite applies to the first body 61.

Figure 6 diagrammatically shows a perspective view in partial cross section of the measuring probe 7' from Figure 5, but without the light conductors 25, 26 and 27, and without the coils, but now with the plate 30, which carries the EIS electrodes 31, a thermometer 65 and the Hall sensors 66.

The function of the thermometer 65 will be clear and that of the EIS electrodes has already been mentioned briefly above. The Hall sensors 66 serve to detect the passing of first body 61 and to determine the fall velocity of the first body from the difference time between passing the successive Hall sensors, according to a technique which is known per se. In this case, the Hall sensors 66 are placed at a mutual distance which increases towards the bottom, so that the measuring resolution is maintained at increasing fall velocity.

Figure 7 diagrammatically shows the first body 61 in perspective. It comprises a tube 70 with a hole 71, as well as single fins 72 with a tapered top side 73. The tube 70 and/or the fins 72 are made from or with a magnetic material. The hole has a cross section which is greater than the width of the plate 30 with the lugs 64. As a result thereof, the first body 61 can move freely from the top to the bottom. The fins 72 have a tapered top side in order to encounter a reduced friction, at least when moving up.

It should be noted here that the illustrated sensor/measuring devices for optical, (di)electrical, viscosity and other properties can be combined as desired in a measuring probe/measuring system according to the invention. In particular in combination with the hot and cold conditioning, if desired with a predeterminable time-temperature variation, valuable information about the products can be collected in an automatic and standardised way, so that, for example, human errors of judgement can be avoided.

By regularly repeating the optical, (di)electrical, viscosity and/or other measurements for different temperatures, times and temperature profiles, it is possible to determine product properties and which products are suitable for certain applications. For example, it is thus possible to determine the product composition which, from a bacterial point of view or otherwise, is most resistant to temperature variations, etc.

In a practical setup, for example as shown in Figure 1, ten modules 2 are each filled with five containers 5 containing one or more products, such as a dairy product. In an example, the simplest time-temperature profile is applied, namely a constant temperature, but with each container having a different temperature. The parameters being measured are the viscosity (at one fall velocity, i.e. without additional "pulling" by the coils), the transmission and diffusion for four wavelengths, and an EIS spectrum. Starting with one measurement per hour, this simplest case already delivers a wealth of information each day (1200 EIS spectra and more than 10,000 other measured values). As all this is done automatically and, based on the measured values, subsequent steps may optionally follow, it is possible to determine a series of product properties in a very efficient way and/or a selection can be made between various products at mutually identical conditions. Such subsequent steps are, for example, automatically indicating if a product no longer meets the product criteria with regard to viscosity, transparency (transmission), etc. The test for the respective product can then be terminated, thus making room for another product, etc.

## Claims

1. Measuring system (1) for automatically determining and/or monitoring the quality of liquid or viscous foodstuffs, and comprising
- a housing (10) with a space (4) for accommodating at least one container (5) for such a liquid or viscous foodstuff,
- at least one container for or with said foodstuff, with a probe (7; 7') which projects into the container,
- a viscosity measuring device for determining a viscosity value of the foodstuff in said space, and
- a control unit (3) which is configured for controlling the measuring system, and which is operatively connected to the viscosity measuring device for repeatedly performing a viscosity measurement and storing and/or exporting and/or processing the determined viscosity value,
wherein the viscosity measuring device comprises:
- a magnetic first body (61),
- a controllable electromagnetic drive (60-1, ..., 60-10) for the first body, and
- a detection system for detecting displacement of the body, comprising a plurality of proximity sensors (66), in particular Hall sensors,
wherein the first body, in use, is displaceable on account of the drive,
wherein, in use, the first body is situated in the container,
wherein the drive comprises a plurality of individually energisable coils (60-1, ..., 60-10) which are wound around the container which are stacked in a stack,
wherein the control unit is configured to individually energise the coils in a predetermined pattern, in such a way that the first body is displaced in the container,
**characterised in that**
the first body, in use, surrounds the probe and is displaceable around the probe, and
the probe comprises a thermometer (65).

2. Measuring system according to Claim 1, wherein the pattern is adjustable.

3. Measuring system according to Claim 1 or 2, wherein the pattern comprises repeatedly energising successive coils in a reciprocating cycle.

4. Measuring system according to Claim 1 or 2, wherein the pattern comprises successively temporarily energising a coil and temporarily energising an adjacent coil after a delay time, in such a way that the first body is displaced in the container at a velocity, wherein the control unit is configured to adjust at least one of the delay time and the velocity.

5. Measuring system according to one of the preceding claims, wherein the plurality of proximity sensors are provided on an elongate carrier (30) in the container.

6. Measuring system according to one of the preceding claims, wherein the plurality of proximity sensors are provided between the container and the plurality of coils.

7. Measuring system according to one of the preceding claims, wherein the plurality of proximity sensors are placed at a mutual distance apart which, in use, increases in a downward direction.

8. Measuring system according to one of the preceding claims, furthermore provided with a cleaning device, comprising a magnetic second body (62) which, in use, is provided in the container under the first body and is displaceable on account of said coils, and is provided with a cleaning part (63) on an outer periphery which bears against an inner wall of the container,
wherein a stop (64) is provided on the carrier and/or on said inner wall of the container for cooperation with the second body, which stop determines a maximum elevation for the second body which is lower than a maximum elevation for the first body.

9. Measuring system according to one of the preceding claims, furthermore comprising a conditioning unit (13 - 18) for conditioning said space of the housing, comprising a heating and cooling device (13, 18), and operatively connected to the thermometer.

10. Measuring system according to Claim 9, wherein the control unit is configured to control the conditioning unit, comprising causing the space of the housing to undergo a predetermined time-temperature program.

## Patentansprüche

1. Messsystem (1) zur automatischen Bestimmung und/oder Überwachung der Qualität von flüssigen oder zähflüssigen Lebensmitteln, umfassend:
- ein Gehäuse (10) mit einem Raum (4) zum Aufnehmen mindestens eines Behälters (5) für ein solches flüssiges oder zähflüssiges Lebensmittel,
- mindestens einen Behälter für oder mit dem Lebensmittel, mit einer Sonde (7; 7'), die in den Behälter hineinragt,
- eine Viskositätsmessvorrichtung zum Bestimmen eines Viskositätswertes des Lebensmittels in diesem Raum und
- eine Steuereinheit (3), die zum Steuern des Messsystems ausgelegt ist und die mit der Viskositätsmessvorrichtung zum wiederholten Durchführen einer Viskositätsmessung und zum Speichern und/oder zum Exportieren und/oder zum Verarbeiten des ermittelten Viskositätswertes in Wirkverbindung steht,
wobei die Viskositätsmessvorrichtung umfasst:
- einen magnetischen ersten Körper (61),
- einen steuerbaren elektromagnetischen Antrieb (60-1, ... , 60-10) für den ersten Körper, und
- ein Erkennungssystem zum Erkennen der Verschiebung des Körpers, das eine Vielzahl von Näherungssensoren (66), insbesondere Hall-Sensoren, umfasst,
wobei der erste Körper beim Gebrauch aufgrund des Antriebs verschiebbar ist,
wobei sich der erste Körper beim Gebrauch in dem Behälter befindet,
wobei der Antrieb eine Vielzahl von einzeln erregbaren Spulen (60-1, ..., 60-10) umfasst, die um den Behälter gewickelt und zu einem Stapel gestapelt sind,
wobei die Steuereinheit dazu ausgelegt sind, die Spulen einzeln in einem vorgegebenen Muster zu erregen, sodass der erste Körper in dem Behälter verschoben wird, **dadurch gekennzeichnet, dass**
der erste Körper beim Gebrauch die Sonde umgibt und um die Sonde herum verschiebbar ist und die Sonde ein Thermometer (65) umfasst.

2. Messsystem gemäß Anspruch 1, wobei das Muster einstellbar ist.

3. Messsystem gemäß Anspruch 1 oder 2, wobei das Muster das wiederholte Erregen aufeinanderfolgender Spulen in einem hin- und hergehenden Zyklus umfasst.

4. Messsystem gemäß Anspruch 1 oder 2, wobei das Muster ein aufeinanderfolgendes temporäres Erregen einer Spule und nach einer Verzögerungszeit ein temporäres Erregen einer benachbarten Spule umfasst, sodass der erste Körper in dem Behälter mit einer Geschwindigkeit verschoben wird, wobei die Steuereinheit dazu ausgelegt ist, mindestens eines von der Verzögerungszeit und der Geschwindigkeit einzustellen.

5. Messsystem gemäß einem der vorhergehenden Ansprüche, wobei die Vielzahl von Näherungssensoren an einem langgestreckten Träger (30) in dem Behälter vorgesehen sind.

6. Messsystem gemäß einem der vorhergehenden Ansprüche, wobei die Vielzahl von Näherungssensoren zwischen dem Behälter und der Vielzahl von Spulen vorgesehen ist.

7. Messsystem gemäß einem der vorhergehenden Ansprüche, wobei die Vielzahl von Näherungssensoren in einem gegenseitigen Abstand zueinander angeordnet sind, der beim Gebrauch nach unten hin zunimmt.

8. Messsystem gemäß einem der vorhergehenden Ansprüche, ferner versehen mit einer Reinigungsvorrichtung, die einen magnetischen zweiten Körper (62) umfasst, der beim Gebrauch in dem Behälter unter dem ersten Körper vorgesehen ist und aufgrund der Spulen verschiebbar ist und an einem Außenumfang mit einem Reinigungsteil (63) versehen ist, der an einer Innenwand des Behälters anliegt,
wobei ein Anschlag (64) an dem Träger und/oder an der Innenwand des Behälters zum Zusammenwirken mit dem zweiten Körper vorgesehen ist, wobei der Anschlag eine maximale Höhe für den zweiten Körper bestimmt, die niedriger als eine maximale Höhe für den ersten Körper ist.

9. Messsystem gemäß einem der vorhergehenden Ansprüche, ferner umfassend eine Konditionierungseinheit (13 - 18) zum Konditionieren des Raums des Gehäuses, die eine Heiz- und Kühlvorrichtung (13, 18) umfasst und mit dem Thermometer in Wirkverbindung steht.

10. Messsystem gemäß Anspruch 9, wobei die Steuereinheit dazu ausgelegt ist, die Konditionierungseinheit zu steuern, indem sie den Raum des Gehäuses veranlasst, ein vorgegebenes Zeit-Temperatur-Programm zu durchlaufen.

## Revendications

1. Système de mesure (1) destiné à déterminer et/ou surveiller automatiquement la qualité de denrées alimentaires liquides ou visqueuses, et comprenant
un boîtier (10) comprenant un espace (4) destiné à loger au moins un récipient (5) pour ces denrées alimentaires liquides ou visqueuses,
au moins un récipient pour ou destiné auxdites denrées alimentaires, doté d'une sonde (7 ; 7') qui se projette dans le récipient,
un dispositif de mesure de viscosité destiné à déterminer une valeur de viscosité des denrées alimentaires dans ledit espace, et
une unité de commande (3) qui est configurée pour commander le système de mesure, et qui est connectée en fonctionnement au dispositif de mesure de viscosité pour exécuter de façon répétée une mesure de la viscosité et stocker et/ou exporter et/ou traiter la valeur de viscosité déterminée,
dans lequel le dispositif de mesure de viscosité comprend :
un premier corps magnétique (61),
un entraînement électromagnétique contrôlable (60-1, ..., 60-10) pour le premier corps, et
un système de détection destiné à détecter un déplacement du corps, comprenant une pluralité de capteurs de proximité (66), en particulier des capteurs à effet Hall,
dans lequel le premier corps, en utilisation, peut être déplacé au moyen de l'entraînement,
dans lequel, en utilisation, le premier corps est situé dans le récipient,
dans lequel l'entraînement comprend une pluralité de bobines pouvant être mises sous tension individuellement (60-1, ..., 60-10) qui sont enroulées autour du récipient, qui sont empilées en pile,
dans lequel l'unité de commande est configurée pour mettre individuellement sous tension les bobines suivant un schéma prédéterminé, de telle sorte que le premier corps soit déplacé dans le récipient,
**caractérisé en ce que**
le premier corps, en utilisation, entoure la sonde et peut être déplacé autour de la sonde, et
la sonde comprend un thermomètre (65).

2. Système de mesure selon la revendication 1, dans lequel le schéma est réglable.

3. Système de mesure selon la revendication 1 ou 2, dans lequel le schéma comprend des bobines successives se mettant sous tension de façon répétée en un cycle de va-et-vient.

4. Système de mesure selon la revendication 1 ou 2, dans lequel le schéma comprend successivement la mise sous tension temporaire d'une bobine et la mise sous tension temporaire d'une bobine adjacente après un délai, de telle sorte que le premier corps soit déplacé dans le récipient à une certaine vitesse, dans lequel l'unité de commande est configurée pour régler au moins l'un du délai et de la vitesse.

5. Système de mesure selon l'une des revendications précédentes, dans lequel les capteurs de la pluralité de capteurs de proximité sont placés sur un support allongé (30) dans le récipient.

6. Système de mesure selon l'une des revendications précédentes, dans lequel les capteurs de la pluralité de capteurs de proximité sont placés entre le récipient et la pluralité de bobines.

7. Système de mesure selon l'une des revendications précédentes, dans lequel les capteurs de la pluralité de capteurs de proximité sont placés à une distance mutuelle qui, en utilisation, augmente dans la direction descendante.

8. Système de mesure selon l'une des revendications précédentes, comprenant en outre un dispositif de nettoyage, comprenant un second corps magnétique (62), qui, en utilisation, est placé dans le récipient sous le premier corps et peut être déplacé au moyen desdites bobines, et est doté d'une partie de nettoyage (63) sur une périphérie externe qui repose contre une paroi interne du récipient,
dans lequel une butée (64) est placée sur le support et/ou sur ladite paroi interne du récipient pour coopérer avec le second corps, laquelle butée détermine une élévation maximale pour le second corps qui est inférieure à une élévation maximale pour le premier corps.

9. Système de mesure selon l'une des revendications précédentes, comprenant en outre une unité de conditionnement (13-18) destinée à conditionner ledit espace du boîtier, comprenant un dispositif de chauffage et de refroidissement (13, 18), et connectée en fonctionnement au thermomètre.

10. Système de mesure selon la revendication 9, dans lequel l'unité de commande est configurée pour commander l'unité de conditionnement, consistant à amener l'espace du boîtier à subir un programme temps-température prédéterminé.
